Europäisches Patentamt

European Patent Office

Office européen des brevets

(11) Publication number: **0 140 255 B1**

(12) **EUROPEAN PATENT SPECIFICATION**

(45) Date of publication of patent specification: **15.05.91**  (51) Int. Cl.⁵: **A61K 9/00, A61K 9/22, A61K 47/00**

(21) Application number: **84112313.6**

(22) Date of filing: **12.10.84**

(54) **Sustained-release injections.**

(30) Priority: **14.10.83 JP 193064/83**
**20.10.83 JP 197181/83**
**01.11.83 JP 206226/83**
**21.11.83 JP 220452/83**

(43) Date of publication of application:
**08.05.85 Bulletin 85/19**

(45) Publication of the grant of the patent:
**15.05.91 Bulletin 91/20**

(84) Designated Contracting States:
**CH DE FR GB LI SE**

(56) References cited:
**BE-A- 882 541**
**FR-A- 2 342 741**
**FR-A- 2 353 285**
**GB-A- 642 385**
**US-A- 2 518 510**

(73) Proprietor: **SUMITOMO PHARMACEUTICALS COMPANY, LIMITED**
**2-8, Dosho-Machi 2-Chome, Chuo-Ku**
**Osaka-Shi Osaka-Fu(JP)**

(72) Inventor: **Yamahira, Yoshiya**
**4-6-7-306, Tanaka-cho Higashinada-ku**
**Kobe-shi Hyogo-ken(JP)**
Inventor: **Fujioka, Keiji**
**2-1, Kuwata-cho**
**Ibaraki-shi Osaka-fu(JP)**
Inventor: **Sato, Shigeji**
**2-29-7, Oike**
**Ibaraki-shi Osaka-fu(JP)**
Inventor: **Yoshida, Noboru**
**1-5-3-618, Higashi-Awaji Higashi-Yodogawa-ku**
**Osaka-shi Osaka-fu(JP)**

(74) Representative: **Vossius & Partner**
**Siebertstrasse 4 P.O. Box 86 07 67**
**W-8000 München 86(DE)**

## Description

The present invention relates to a sustained-release injection preparation. More particularly, it relates to a sustained-release injection preparation which comprises a suspension of a powder comprising an active ingredient and a pharmaceutically acceptable biodegradable carrier in a viscous suspension medium for injection preparation.

It is known that a sustained-release injection preparation is prepared by dissolving or suspending an active ingredient in polyethylene glycol, an oil for injection or gelatin solution, but these known preparations are not satisfactory and can not be applied to a water-soluble medicament which is effective in a small amount. Besides, although a preparation using a non-biodegradable carrier such as silicone has been recently used as a sustained-release preparation in some medical sections, it is not preferable because when it is administered in a parenteral route, there is a problem of accumulation of the carrier.

It has been very desirable to make various medicaments in the form of a sustained-release injection preparation, because the pharmaceutical activities of the medicaments will be potentiated in such a preparation, and hence, it is useful to develop a sustained-release injection preparation of medicaments.

FR-A-2 342 741 describes a composition containing a biodegradable matrix and a biologically active substance which is incorporated into the matrix. The matrix consists of gelatine which may be cross-linked with a dialdehyde whereby only the composition containing cross-linked gelatine exhibits sustained-release properties.

FR-A-2 353 285 discloses a medicament comprising an active ingredient enclosed in a film of a water-insoluble material, e.g. gelatine, which medicament may be in the form of grains dispersed in a liquid.

The present inventors made intensive studies regarding an improved sustained-release injection preparation of medicaments, and have found that the desired sustained-release injection preparation can be obtained by admixing an active ingredient with a specific biodegradable carrier and suspending the powdery mixture in a viscous suspension medium for injection preparation.

An object of the present invention is to provide an improved sustained-release injection preparation. Another object of the invention is to provide a suspension type, sustained-release injection preparation which can release the active ingredient and can maintain the desired level of the active ingredient in blood or in lesional regions for a long period of time. These and other objects and advantages of the present invention will be apparent to persons skilled in the art from the following description.

The sustained-release injection preparation of the present invention comprises a suspension of a powdery mixture in a viscous suspension medium for injection preparation, said powdery mixture comprising a pharmaceutically acceptable biodegradable carrier selected from collagen, atelocollagen, a mixture of them, and a mixture thereof with gelatin and a usual amount of a pharmaceutically active ingredient incorporated into said carrier.

The biodegradable carrier used in the present invention means a carrier which can easily be absorbed or be subject to enzymolysis in body and can be implanted into the body. Suitable examples of the biodegradable carrier are proteins such as collagen, atelo collagen or a mixture of them or a mixture thereof with gelatin. Collagen is a protein which is a main protein of connective tissue of animals and has less antigenicity, and hence, has widely been used as a safe operation yarn in various medical operations. Atelocollagen has far less antigenicity which is obtained by removing the telopeptide region by treating collagen with an enzyme (e.g. pepsin) in order to make it safer. Gelatin is a protein derived from collagen. Gelatin is a high molecular weight amphoteric electrolyte which has less antigenicity and can be converted from the sol form to the gel form and is low in cost. Hence it has already been confirmed as a safe substance for medical use.

The active ingredient used in the present invention is not specified, but includes particularly water-soluble medicaments which are hardly prepared in the form of a sustained-release injection by a conventional method, for example, prostaglandins, prostacyclines, various bio-hormones, bleomycins, mitomycin, tespamin (triethylenethiophosphoramide), interferon, interleukin, tumor necrosis factor, and further hardly water-soluble medicaments which are effective in a very small amount, such as indomethacin, adriamycin, or the like. The medicament includes also 4-carbamoyl-imidazolium-5-oleate (other name: 4-carbamoyl-5-hydroxyimidazole or SM-108) or a salt or a hydrate thereof, which compound (and its salts and hydrates) is known as a purine antitumor agent and is effective as a metabolic antagonist in purine de novo synthesis.

Among the above medicaments, prostaglandins, prostacyclines, various bio-hormones, mitomycin, tespamine, interferon, interleukin, and tumor necrosis factor are very unstable not only within a body but also in the form of a preparation, and hence the activity thereof is largely and rapidly decreased by the conventional release-sustaining techniques such as heat treatment or irradiation, or by chemical treatment with organic solvents or aldehydes. Moreover, since these medicaments are water-soluble and are used in a very small amount, it is very difficult to prepare sustained-release preparations thereof by a conventional method. Various interferons, interleukins and tumor necrosis factor are somewhat different to each other, but they have in common that they have a very similar molecular weight and are glycoproteins or proteins and have similar pharmacological and physicochemical properties to those of $\alpha$-interferon, as shown in the experiments disclosed hereinafter, and all of these compounds are prepared in the form of desired excellent sustained-release injection preparations according to the present invention.

The viscous suspension media used for suspending the powder of the active ingredient and the biodegradable carrier include all conventional viscous media for injection, such as vegetable oils, polyethylene glycol, propylene glycol, silicone oil, medium-chain fatty acid triglycerides, or the like. Suitable examples of the vegetable oils are peanut oil, cotton seed oil, sesame oil, castor oil, olive oil, corn oil, iodinated poppy seed oil fatty acids ethyl esters, or the like.

The preparation of the present invention contains the active ingredient in an amount in which the active ingredient is usually used. For example, indomethacin is usually contained in an amount of 0.5 to 500 mg, preferably 1 to 200 mg, per dosage unit, and interferon is usually contained in an amount of $10^4$ to $10^9$ IU, preferably $10^5$ to $5 \times 10^8$ IU, per dosage unit, and SM-108 or a salt or hydrate thereof is usually contained in an amount of 1 mg to 2 g, preferably 10 mg to 1 g, per dosage unit.

Besides, the ratio of the medicament and the carrier is not specified, but, for example, indomethacin is preferably incorporated in an amount of 0.005 to 1 mg per 1 mg of the carrier, and interferon is preferably incorporated in an amount of $10^3$ to $10^8$ IU per 1 mg of the carrier, and SM-108 is preferably incorporated in an amount of 0.01 to 1 mg per 1 mg of the carrier.

The sustained-release injection preparation of the present invention is prepared in the following manner.

Firstly, a powder of an active ingredient contained in a biodegradable carrier is prepared, and the powder is suspended in a viscous suspension medium for injection preparation. The preparation of the powder can be done by any method by which the active ingredient is incorporated in the carrier, for example, by admixing the active ingredient in an aqueous solution of the biodegradable carrier, lyophilizing the mixture and then pulverizing. Since the pulverizing step is usually exothermic, some medicaments such as interferon are preferably treated under cooling with dry ice or liquid nitrogen. Spray drying is also applicable.

The pulverized product having an injectable particle size (e.g. 0.1 to 1000 $\mu$m) is then suspended in a viscous suspension medium for injection preparation to give a sustained-release suspension for injection preparation. Alternatively, the pulverized product and the viscous suspension medium may be packed in the form of a kit, and both ingredients are mixed to prepare a suspension for injection when used.

In the sustained-release injection preparation of the present invention, the active ingredient is incorporated into the biodegradable carrier in the following state:

(i) the active ingredient is chemically bound to the carrier matrix,

(ii) The active ingredient is bound to the carrier matrix by intermolecular action, or

(iii) The active ingredient is physically embraced within the carrier matrix.

In the preparation, there may optionally be incorporated conventional pharmaceutically acceptable additives such as stabilizers, preservatives, local anesthetic agents, and some agents for aiding formability into special shapes of preparations or release-sustaining of the active ingredient. These additives are not specified, but suitable examples of the agents for aiding formability are methylcellulose, ethylcellulose, hydroxypropylcellulose, polyvinylpyrrolidone, glycolic acid-lactic acid copolymer, polyethylene glycol, propylene glycol, ethyl alcohol, or the like. Suitable examples of the agents for aiding release-sustaining of the active ingredient are cellulose acetate phthalate, ethylcellulose, methylcellulose, hydroxypropylmethylcellulose, calcium phosphate, lactic acid-glycolicacid copolymer, corn starch, rice starch, potato starch, cellulose, arginates, or the like.

All steps for the above preparation should be carried out under sterilized conditions because the preparations are used as an injection preparation.

Thus, according to the present invention, the medicaments which are water-soluble and effective in a very small amount are embraced within a carrier which has affinity with the active ingredient and then are suspended in a viscous suspension medium suitable for injection in the form of a powder, by which there is prepared a sustained-release preparation suitable for injection, which is the novel form of an injection preparation.

The present invention is illustrated by the following Experiment and Examples, but should not be construed to be limited thereto.

Experiment 1

There were used as the test samples an oily suspension of α-interferon-collagen preparation prepared in Example 1 disclosed hereinafter (Sample a) and an aqueous injection of α-interferon (originated from Namalwa cells) (sample b) as a reference. The test samples were each administered intramuscularly to rabbit, and the change of level in blood of the active ingredient with lapse of time was measured by RIA (radioimmunoassay). Two rabbits were used for each sample, and the test samples were each administered in a dose of $10^6$ U/kg. The blood level was measured in average in two rabbits.

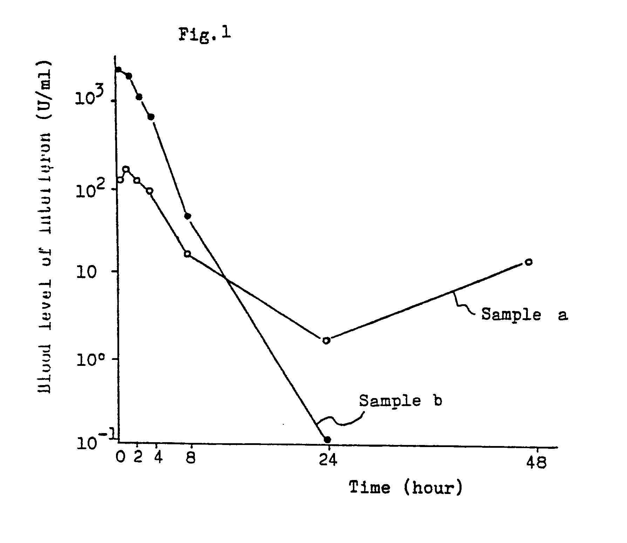

The results are shown in the accompanying Fig. 1. In Fig. 1, o is the graph of Sample a and ● is that of Sample b (α-interferon aqueous injection). As is clear from the figure, sample a showed release-sustaining, and even after 48 hours, the blood level of several tens unit/ml was maintained.

Thus, it is also suggested by the test of in vivo using rabbits that the preparation of the present invention is useful clinically.

Example 1

An aqueous solution of α-interferon (titer: 4.9 MU/ml) (100 ml) and 2 % atelocollagen (50 g) is homogeneously mixed while stirring, producing as little foam as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial (Sample a).

Example 2

An aqueous solution of α-interferon (titer: 4.9 MU/ml) (100 ml) and 2 % collagen (50 g) is homogeneously mixed while stirring, producing as little foam as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial.

Example 3

An aqueous solution of α-interferon (titer, 4.9 MU/ml) (100 ml), 2 % atelocollagen (50 g), human serum albumin (150 mg) and thimerosal (120 µg) is homogeneously mixed producing as little foam as possible. The mixture is lyophilized and pulverized at a low temperature using liquid nitrogen. The pulverized product thus obtained is suspended in sesame oil to give an oily suspension type, sustained-release preparation wherein interferon is contained in an amount of 4 MU per 1 vial.

Example 4

The pulverized product prepared in the same manner as described in Example 1 is suspended in castor oil to give an oily suspension type, sustained-release preparation wherein interferon is contained in an amount of 4 MU per 1 vial.

Example 5

The pulverized product prepared in the same manner as described in Example 1 is suspended in polyethylene glycol to give a suspension type, sustained-release preparation wherein interferon is contained in an amount of 4 MU per 1 vial.

Example 6

An aqueous solution of α-interferon (titer, 4.9 MU/ml) (100 ml), 2 % atelocollagen (50 g) and tespamin (triethylenethiophosphoramide, which is known as an antineoplastic) (245 mg) is homogeneously mixed producing as little foam as possible. The mixture is lyophilized and pulverized at a low temperature using liquid nitrogen. The pulverized product thus obtained is suspended in sesame oil to give an oily suspension type, sustained-release preparation wherein interferon and tespamin are contained in an amount of 4 MU and about 2 mg per 1 vial, respectively.

Example 7

The pulverized product prepared in the same manner as described in Example 1 is suspended in iodinated poppy seed oil fatty acids ethyl esters (sold by Libiodol Ultra-fluid - Kodama Shoji) to give an oily suspension type, sustained-release preparation wherein interferon is contained in an amount of 4 MU per 1 vial.

Example 8

2 % Atelocollagen (75 g) is dissolved in distilled water (300 ml), and thereto are added indomethacin (0.5 g) and arginine (0.292 g). The mixture is lyophilized and pulverized at a low temperature using liquid nitrogen. The pulverized product thus obtained is suspended in sesame oil to give an oily suspension type, sustained-release preparation.

Example 9

2 % atelocollagen (100 g) is dissolved in distilled water (1 liter), and thereto are added SM-108 hydrate (2 g) and sodium hydrogensulfite (100 mg). The mixture is filtered and then lyophilized. The lyophilized product thus obtained is pulverized at a low temperature, and the pulverized product is suspended in sesame oil to give an oily suspension type, sustained-release preparation.

## Claims

1. A sustained-release injection preparation, which comprises a suspension of a powdery mixture in a viscous suspension medium for injection preparation, said powdery mixture comprising a pharmaceutically acceptable biodegradable carrier selected from collagen, atelocollagen, a mixture of them, and a mixture thereof with gelatin and a usual amount of a pharmaceutically active ingredient incorporated into said carrier.

2. The preparation according to claim 1, wherein the pharmaceutically active ingredient is a water-soluble or a hardly water-soluble medicament which is effective in a very small amount.

3. The preparation according to claim 1 or 2, wherein the viscous suspension medium for injection preparation is a member selected from vegetable oils, polyethylene glycol, propylene glycol, silicone oil, and medium-chain fatty acid triglycerides.

4. The preparation according to claim 1, wherein the viscous suspension medium for injection preparation is a member selected from peanut oil, cotton seed oil, sesame oil, castor oil, olive oil, corn oil, and iodinated poppy seed oil fatty acids ethyl esters.

5. The preparation according to claim 1, wherein the viscous suspension medium is sesame oil.

6. The preparation according to claim 1, wherein the active ingredient is incorporated into the biodegradable carrier in the following state:

(i) the active ingredient is chemically bound to the carrier matrix,
(ii) the active ingredient is bound to the carrier matrix by intermolecular action, or
(iii) the active ingredient is physically embraced within the carrier matrix.

7. The preparation according to claim 1, wherein the active ingredient is a member selected from indomethacin, prostaglandins, prostacyclines, bio-hormones, tespamin, adriamycin, bleomycins, mitomycin, interferon, interleukin, tumor necrosis factor, and 4-carbamoyl-imidazolium-5-oleate or a salt or hydrate thereof.

8. The preparation according to claim 1, wherein the active ingredient is a member selected from interferon, interleukin and tumor necrosis factor.

9. The preparation according to claim 1, wherein the active ingredient is interferon.

10. A method for the preparation of a sustained-release injection preparation according to claims 1-9 which comprises mixing a usual amount of an active ingredient and a pharmaceutically acceptable biodegradable carrier to incorporate the active ingredient in to the carrier matrix, pulverizing the carrier matrix, and then suspending the pulverized product in a viscous suspension medium for injection preparation.

## Revendications

1. Une préparation injectable à libération prolongée, qui comprend une suspension d'un mélange en poudre dans un milieu de suspension visqueux pour préparation injectable, ledit mélange en poudre comprenant un support biodégradable pharmaceutiquement acceptable choisi parmi le collagène, l'atélocollagène, un de leurs mélanges et un de leurs mélanges avec la gélatine et une quantité habituelle d'un ingrédient pharmaceutiquement actif incorporée dans ledit support.

2. La préparation selon la revendication 1, dans laquelle l'ingrédient actif est un médicament soluble dans l'eau ou un médicament peu soluble dans l'eau qui est efficace en très faible quantité.

3. La préparation selon la revendication 1 ou 2, dans laquelle le milieu de suspension visqueux pour préparation injectable est choisi parmi les huiles végétales, le polyéthylèneglycol, le propylèneglycol, les huiles de silicones et les triglycérides d'acides gras à chaîne moyenne.

4. La préparation selon la revendication 1, dans laquelle le milieu de suspension visqueux pour préparation injectable est choisi parmi l'huile d'arachide, l'huile de graines de coton, l'huile de sésame, l'huile de ricin, l'huile d'olive, l'huile de maïs et les esters éthyliques d'acides gras d'huile de graines de pavot iodée.

5. La préparation selon la revendication 1, dans laquelle le milieu de suspension visqueux est l'huile de sésame.

6. La préparation selon la revendication 1, dans laquelle l'ingrédient actif est incorporé dans le support biodégradable à l'état suivant :
   (i) l'ingrédient actif est chimiquement lié à la matrice de support,
   (ii) l'ingrédient actif est lié à la matrice de support par une action intermoléculaire, ou bien
   (iii) l'ingrédient actif est physiquement enfermé dans la matrice de support.

7. La préparation selon la revendication 1, dans laquelle l'ingrédient actif est choisi parmi l'indométhacine, les protaglandines, les prostacyclines, les biohormones, la tespamine, l'adriamycine, les bléomycines, la mitomycine, l'interféron, l'interleukine, le facteur de nécrose des tumeurs et le 4-carbamoyl-imidazolium-5-oléate ou ses sels ou hydrates.

8. La préparation selon la revendication 1, dans laquelle l'ingrédient actif est choisi parmi l'interféron, l'interleukine et le facteur de nécrose des tumeurs.

9. La préparation selon la revendication 1, dans laquelle l'ingrédient actif est l'interféron.

10. Un procédé pour la préparation d'une préparation injectable à libération prolongée selon les revendications 1 à 9, qui consiste à mélanger une quantité habituelle d'un ingrédient actif et un support biodégradable pharmaceutiquement acceptable pour incorporer l'ingrédient actif dans la matrice de support, à pulvériser la matrice de support et ensuite à mettre en suspension le produit pulvérisé dans un milieu de suspension visqueux pour préparation injectable.

**Ansprüche**

1. Injektionspräparat mit Langzeitwirkung, das eine Suspension eines pulverförmigen Gemisches in einem viskosen Suspensionsmedium für ein Injektionspräparat enthält, wobei das pulverförmige Gemisch einen pharmakologisch verträglichen, biologisch abbaubaren Träger, nämlich Kollagen, Atelokollagen, ein Gemisch davon, oder ein Gemisch davon mit Gelatine und eine übliche Menge eines in den Träger aufgenommenen pharmakologisch aktiven Wirkstoffs enthält.

2. Präparat nach Anspruch 1, worin der pharmakologisch aktive Wirkstoff ein in äußerst geringer Menge wirksames wasserlösliches oder schwer wasserlösliches Medikament ist.

3. Präparat nach Anspruch 1 oder 2, bei dem das viskose Suspensionsmedium für das Injektionspräparat ein Pflanzenöl, Polyethylenglykol, Propylenglykol, Silikonöl oder ein mittellanges Fettsäuretriglycerid ist.

4. Präparat nach Anspruch 1, bei dem das viskose Suspensionsmedium für das Injektionspräparat Erdnußöl, Baumwollsamenöl, Sesamöl, Rizinusöl, Ölivenöl, Maisöl oder Fettsäureethylester von jodiertem Mohnsamenöl ist.

5. Präparat nach Anspruch 1, bei dem das viskose Suspensionsmedium Sesamöl ist.

6. Präparat nach Anspruch 1, worin der Wirkstoff in den biologisch abbaubaren Träger in folgendem Zustand aufgenommen wird:
   (i) der Wirkstoff wird chemisch an die Trägermatrix gebunden,
   (ii) der Wirkstoff wird an die Trägermatrix durch intermolekulare Wechselwirkung gebunden, oder
   (iii) der Wirkstoff wird physikalisch in der Trägermatrix festgehalten.

7. Präparat nach Anspruch 1, worin der Wirkstoff Indomethacin, ein Prostaglandin, ein Prostacyclin, ein Biohormon, Tespamin, Adriamycin, Bleomycin, Mitomycin, Interferon, Interleukin, Tumornekrosefaktor, 4-Carbamoyl-imidazolium-5-Oleat oder ein Salz oder Hydrat davon ist.

8. Präparat nach Anspruch 1, worin der Wirkstoff Interferon, Interleukin oder Tumornekrosefaktor ist.

9. Präparat nach Anspruch 1, worin der Wirkstoff Interferon ist.

**10.** Verfahren zur Herstellung eines Injektionspräparates mit Langzeitwirkung nach den Ansprüchen 1 bis 9, das das Mischen einer üblichen Menge eines Wirkstoffes mit einem pharmakologisch verträglichen biologisch abbaubaren Träger umfaßt, um den Wirkstoff in die Trägermatrix aufzunehmen, das Pulverisieren der Trägermatrix, und anschließend das Suspendieren des pulverisierten Produkts in einem viskosen Suspensionsmedium für das Injektionspräparat.

Fig. 1